# EUROPEAN PATENT APPLICATION

(11) **EP 3 903 807 A1**
(43) Date of publication of application: **03.11.2021**
(21) Application number: 21742294.8
(22) Date of filing: 10.03.2021
(51) Int. Cl.: A61K 38/18

(54) **COMPOSITION FOR TREATING HAIR LOSS OR PROMOTING HAIR GROWTH COMPRISING GROWTH FACTOR**

(30) Priority: 11.03.2020 KR 20200029994
(71) Applicant: MEDICOSBIOTECH INC., Daejeon 34141 (KR)
(72) Inventor: YOO, Won Min, Gangnam-gu, Seoul 06293 (KR); CHUNG, Hannah, Yuseong-gu, Daejeon 34127 (KR); NA, Deok Hyun, Goyang-si, Gyeonggi-do 10304 (KR)
(74) Representative: Mewburn Ellis LLP
(86) International application number: PCT/KR2021/002969
(87) International publication number: WO 2021/182866

(57) **Abstract**

The present invention is related to a composition for promoting hair growth or inhibiting hair loss comprising a growth factor selected from the group consisting of VEGF, FGFα, FGFβ, TGF-β, IGF, KGF, and SCF, as an active ingredient, according to the present invention, there is an effect of promoting hair growth and preventing hair loss by regulating the hair cycle of patients with hair loss, without causing side effects such as skin irritation and the like.

SEQUENCE LISTING

<110>  MEDICOSBIOTECH, INC.

<120>  Composition for Treating Hair Loss or Promoting Hair
       Growth Comprising Growth Factor

<130>  008098857

<140>  EP
<141>  2021-03-10

<150>  PCT/KR2021/002969
<151>  2021-03-10

<150>  KR 10-2020-0029994
<151>  2020-03-11

<160>  1

<170>  PatentIn version 3.5


<210>  1
<211>  35
<212>  PRT
<213>  Artificial Sequence

<220>
<223>  recombinant silk protein

<400>  1

Ser Gly Arg Gly Gly Leu Gly Gly Gln Gly Ala Gly Met Ala Ala Ala
1               5                   10                  15


Ala Ala Met Gly Gly Ala Gly Gln Gly Gly Tyr Gly Gly Leu Gly Ser
            20                  25                  30


Gln Gly Thr
        35

## Description

### Technical Field

The present invention relates to a composition for treating hair loss or promoting hair growth comprising a growth factor, and more particularly to a composition for promoting hair growth or inhibiting hair loss comprising a growth factor selected from the group consisting of VEGF, FGFα, FGFβ, TGF-β, IGF, KGF, and SCF as an active ingredient.

### Background Art

Hair performs the function of protecting the scalp from UV rays and external stimuli, and plays an important role in aesthetics. Hair loss afflicts men and some women, and in modern society, the number of patients with hair loss is increasing due to environmental pollution, stress, and a poor work-life balance. The most common type of hair loss in men is male pattern baldness or alopecia. In the case of general alopecia, hair loss occurs gradually over several years, and begins most clearly at the crown of the head and progresses toward the forehead area. For women suffering from alopecia, hair loss occurs in a more dispersed pattern while the hair becomes thinner, compared to alopecia in men, and often appears after menopause.

Hair constantly undergoes a hair cycle including anagen, catagen, telogen, and exogen phases. In order to prevent hair loss, it is necessary to control this hair cycle.

Many candidate compounds have been developed in conventional attempts to produce hair growth promoters. A variety of documents, including medical, scientific and patent documents, show that diverse efforts have been made to treat and/or prevent hair loss and restore and/or promote hair growth, particularly with regard to hair on the human scalp. Indeed, many different active compounds have been proposed, representative examples of which include 2,4-diamino-6-piperidinopyrimidine-3-oxide (also known as "minoxidil") and finasteride which is a specific inhibitor of type II reductase (U.S. Patent Nos. 4,139,619 and 4,596,812). A medicament using minoxidil as an active ingredient is commercially available as Rogaine (a trademark of Pharmacia & Upjohn Company). Rogaine has been reported to reduce hair loss by up to 10% and to promote hair growth in men suffering from male pattern baldness. It is known that Rogaine is a solution that is applied directly onto the scalp area, and also that the therapeutic application thereof has to be continued regularly over a long period of time. A medicament using finasteride as an active ingredient is commercially available as Propecia (a trademark of Merck & Co., Inc.). Propecia is a pill for oral administration, and also has to be administered in a continuous and regular manner.

Recently, the possible effectiveness of animal-derived serum or plasma in promoting hair growth has been suggested (Korean Patent No. 0617644). Meanwhile, it has been suggested that many compositions based on natural plant extracts, including medicinal herbs, may be used for the treatment of alopecia. Several extracts of drugs commonly known as hair growth compositions are used as hair growth stimulants or promoters. In reality, however, these extracts usually do not exert a positive effect on hair growth because the condition of the hair or alopecia varies from person to person. Although some such compositions for hair growth exhibit a predetermined effect, these are accompanied by defects that may cause difficulties in continuous use over a long period of time due to skin irritation, unpleasant odors, and the like.

An alternative to treating alopecia is hair transplantation. This method typically includes implanting natural hair from an area of the scalp in which hair is growing to a bald area. However, hair transplants often detach about 2-4 weeks after implantation. Although most transplants regrow after 3-4 months, additional transplantation is frequently necessary. Hence, this transplantation method is expensive, time-consuming, and painful, and has a relatively low probability of success. Accordingly, there is an urgent need to develop a method and composition for hair growth capable of exhibiting excellent effects of treating alopecia or promoting hair growth over a shorter period of time, without causing side effects.

Therefore, the present inventors have made great efforts to develop a composition having an excellent hair-loss treatment effect and hair growth effect, and thus ascertained that a composition comprising a growth factor and a silk protein has activity of inducing and promoting hair growth in patients with alopecia. Based on this finding, the present invention has been completed.

### Summary of the Invention

It is an object of the present invention to provide a composition for promoting hair growth or inhibiting hair loss, which exhibits activity of inducing and promoting hair growth in patients with alopecia, without causing side effects.

It is another object of the present invention to provide a method of promoting hair growth or inhibiting hair loss, which exhibits activity of inducing and promoting hair growth of patients with alopecia, without causing side effects.

In order to achieve the above objects, the present invention provides a pharmaceutical composition for promoting hair growth or inhibiting hair loss comprising a growth factor selected from the group consisting of VEGF, FGFα, FGFβ, TGF-β, IGF, KGF, and SCF as an active ingredient.

In addition, the present invention provides a pharmaceutical composition for promoting hair growth or inhibiting hair loss comprising VEGF, FGFα, FGFβ, TGF-β, IGF, KGF, and SCF as active ingredients.

In addition, the present invention provides a cosmetic composition for promoting hair growth or inhibiting hair loss comprising a growth factor selected from the group consisting of VEGF, FGFα, FGFβ, TGF-β, IGF, KGF, and SCF as an active ingredient.

In addition, the present invention provides a cosmetic for promoting hair growth or inhibiting hair loss comprising a growth factor selected from the group consisting of VEGF, FGFα, FGFβ, TGF-β, IGF, KGF, and SCF as an active ingredient.

In addition, the present invention provides a method of promoting hair growth or inhibiting hair loss comprising administering a growth factor selected from the group consisting of VEGF, FGFα, FGFβ, TGF-β, IGF, KGF, and SCF.

In addition, the present invention provides the use of a growth factor selected from the group consisting of VEGF, FGFα, FGFβ, TGF-β, IGF, KGF, and SCF for the promotion of hair growth or inhibition of hair loss.

In addition, the present invention provides the use of a growth factor selected from the group consisting of VEGF, FGFα, FGFβ, TGF-β, IGF, KGF, and SCF for the preparation of a therapeutic agent for the promotion of hair growth or inhibition of hair loss.

### Brief Description of Drawings

FIG. 1 shows the results of a scratch assay of NIH3T3 cells, which are fibroblasts, depending on the treatment of growth factors.
FIG. 2 shows the results of a scratch assay of NIH3T3 cells, which are fibroblasts, depending on the treatment of VEGF and a silk protein.
FIG. 3 shows results confirming the state of hair growth in mouse models on the 3^{rd} and 7^{th} days after treatment of an essence containing seven growth factors and an essence containing growth factors and a silk protein to hair-loss mouse models.

### Detailed Description and Preferred Embodiments of the Invention

Unless otherwise defined, all technical and scientific terms used herein have the same meanings as those typically understood by those skilled in the art to which the present invention belongs. Generally, the nomenclature used herein is well known in the art and is ordinarily used.

In the present invention, when a composition comprising a growth factor selected from the group consisting of VEGF, FGFα, FGFβ, TGF-β, IGF, KGF, and SCF is applied onto depilated mouse skin, it is confirmed that a superior hair growth effect is exhibited, and also, when a composition comprising a silk protein in addition to the growth factor is applied onto the depilated mouse skin, it is confirmed that a vastly superior hair growth effect is exhibited.

Accordingly, in one aspect, the present invention is directed to a pharmaceutical composition for promoting hair growth or inhibiting hair loss comprising a growth factor selected from the group consisting of VEGF, FGFα, FGFβ, TGF-β, IGF, KGF, and SCF as an active ingredient.

As used herein, the term "inhibiting hair loss" refers to preventing, inhibiting, suppressing, and reducing partial or complete hair loss.

As used herein, the term "hair growth" is defined as including all of maintenance, induction, stimulation, promotion, and regeneration of hair development in mammals, growth of defective hair, extension of the anagen phase in the hair cycle, and the conversion of vellus hair into terminal hair.

As used herein, the term "alopecia" refers to defective hair growth and partial or complete loss of hair, and examples thereof include, but are not limited to, androgenetic alopecia or male pattern baldness, toxic alopecia, alopecia areata, telogen effluvium, alopecia caused by endocrine abnormalities, metabolic disorders or nutritional disorders, drug-induced alopecia, alopecia mechanica, alopecia caused by skin disease, scarring alopecia, congenital alopecia, and trichotillomania. Alopecia occurs when the hair cycle (pilar cycle) is disrupted. The most frequent phenomenon is shortening of the hair growth or anagen phase due to cessation of cell proliferation. This leads to the early onset of the catagen phase, and ultimately a number of hairs enter the telogen phase. During the telogen phase, the hair follicles detach from the dermal papilla and the hair falls out. Alopecia has many etiologies, including genetic factors, aging, local and systemic diseases, fever symptoms, mental stress, hormonal problems, and side effects of drugs.

As used herein, the term "treating alopecia" refers to preventing alopecia, inhibiting, retarding or reducing alopecia, promoting hair growth, prolonging the anagen phase of the hair cycle, and/or converting vellus hair into terminal hair, in any animals capable of developing alopecia. Here, terminal hair is thick, pigmented, long hair in which hair follicle roots sit deep within the dermis, whereas vellus hair is thin, smooth, short, unpigmented hair in which hair follicle roots sit on the surface of the dermis. With the progression of alopecia, the hair changes from a terminal hair type to a vellus hair type.

The "growth factor" of the present invention is a signal transmitter necessary for the process of making healthy cells through cell division, is composed of a protein, plays a role in continuously growing new skin through growth and differentiation of skin cells, and is also present in the reproductive organs, brain, and nerve cells. Moreover, the growth factor is present in the blood but the amount thereof is insignificant, and it is made of a recombinant protein and is useful as a material for cosmetics.

In the present invention, the growth factor is known to activate hair germinal matrix cells and dermal papilla cells and promote the division of hair root cells, thereby making it possible to expand and form scalp capillaries, thus supplying nutrients to the hair and promoting hair growth. With regard to the application of a growth factor, the effect thereof is maximized in the presence of hair follicles, and for most patients with hair loss in the state in which hair roots are in the telogen phase, the cells are stimulated and activated, thus alleviating hair loss. Examples of the growth factor that may be used in the present invention may include VEGF, FGFα, FGFβ, TGF-β, IGF, KGF, and SCF.

In the present invention, VEGF (vascular endothelial growth factor; sh-polypeptide-9) is a factor that promotes the proliferation of cells inside blood vessels, and induces angiogenesis, thereby increasing the size and distribution of the capillaries of the hair follicles to thus enable cell growth through vascular development and support. Moreover, it is able to control the formation of blood vessels around the hair follicles during the hair cycle and to promote hair regeneration after hair loss. Furthermore, VEGF induces cell migration, physiologically lowers blood pressure, increases the solubility of microvessels, and may be used as an important factor for anti-aging, regeneration of damaged skin, and regeneration and growth of hair.

In the present invention, FGFα (acidic fibroblast growth factor; sh-polypeptide-11) promotes the growth of epidermal cells during wound healing, helps to regenerate hair follicles by participating in collagen synthesis, and stimulates keratinocytes and fibroblasts constituting the skin to regenerate the skin and restore a healthy skin condition.

In the present invention, FGFβ (basic fibroblast growth factor; sh-polypeptide-1) is involved in hair follicle differentiation, collagen production, angiogenesis, and fibroblast activity, and helps to synthesize the extracellular matrix (ECM), such as collagen, elastin, etc. to promote the growth of hair and skin cells, activation of dermal papilla cells, and angiogenesis, thereby stimulating the anagen phase of hair growth. Moreover, it protects the skin from damage caused by UV rays and helps to regenerate the epidermis.

In the present invention, TGF-β (transforming growth factor beta 1; rh-polypeptide-22) stimulates the growth of epidermal cells during wound healing, promotes the synthesis of collagen, promotes the formation of hair follicles so that hair grows, and plays a role in promoting tissue regeneration and recovery using various growth factors and cytokines in the process of healing wounds on the skin and the scalp.

In the present invention, IGF (insulin-like growth factor 1; sh-oligopeptide-2) is a growth factor called somatomedin C, and is also known as a sulfation factor that promotes the production of a growth hormone. Similarly to the growth hormone, IGF contributes to cell growth, improves angiogenesis and the ability to regenerate hair germinal matrix cells, promotes the proliferation of keratinocytes, and regulates the growth of hair follicles, thus contributing to hair growth, thereby promoting the formation of hair matrix cells, keratogenesis, and cell division. Moreover, it delays the entry of hair into the telogen phase, helps to extend the anagen phase, helps to form healthy hair, and prevents hair damage and hair loss. Further, in association with other growth factors, IGF helps in the growth and division of skin cells, helps in the production of ECM substances such as collagen, fibronectin, elastin and keratin in the dermis to slow aging and wrinkling, and is involved in ECM production. Furthermore, because insulin-deficient hair enters the catagen phase, administration of IGF makes it possible to maintain the hair in the anagen phase and is effective at increasing PDGFα/β involved in hair growth.

In the present invention, KGF (keratinocyte growth factor; rh-polypeptide-3) is a major growth factor that stimulates the differentiation, growth and physiological activity of keratinocytes constituting the keratin of the skin, and in the course of wound healing, promotes the division of skin tissue and epidermalization, and helps to complete wound healing. KGF interacts with EGF, IGF, FGFα, and FGFβ to prevent skin aging, is essential for the recovery of wounded areas, helps in the production of endothelial cells and hair, and is known to increase TGF-β1 and to have a UV-blocking effect.

In the present invention, SCF (stem cell factor; sh-polypeptide-4) is a factor that regulates the differentiation of stem cells and progenitor cells, and is known as a kit-ligan, KL or steel factor. It is a major growth factor that binds to the c-Kit receptor (CD117) and exhibits physiological regulatory action. Moreover, SCF is an essential element that is involved in blood formation, spermatogenesis and melanin formation and activates stem cells present in almost all tissues including the skin, and is effective at preventing aging in the skin, and, in the hair, promoting the formation of new hair follicles and slowing the graying of hair.

In the present invention, the growth factor may comprise at least two growth factors selected from the group consisting of VEGF, FGFα, FGFβ, TGF-β, IGF, KGF, and SCF.

The composition of the present invention may further comprise a silk protein.

In the present invention, the silk protein may be naturally derived, or be derived from a recombinant silk protein, and the silk protein may be a repeating unit peptide constituting a protein selected from the group consisting of dragline silk, elastin, silk fibroin, byssus, sericin, flagelliform silk, and collagen.

In the present invention, the silk protein may have a structure in which a silk repeating unit peptide having a glycine or serine content of 1% or more is repeated 1 to 160 times, and the silk protein may have a molecular weight of 5 to 300 kDa.

Thorough research into the use of silk proteins for various biochemical and medicinal applications has been conducted recently, and thus silk proteins are receiving attention as a new biomaterial. In the present invention, a silk protein and a silk peptide have excellent moisturizing ability and good adsorption to keratin, and thus it is confirmed that when the composition for promoting hair growth and preventing hair loss comprises the same, an effect of making the hair appear healthy is exhibited.

The composition of the present invention may be manufactured in a formulation selected from the group consisting of an essence, shampoo, cream, lotion, tonic, spray, aerosol, oil, solution, suspension, gel, and ointment.

In order to effectively treat alopecia or to effectively promote hair growth, it must be easy for the growth factor and/or silk protein used in the method and pharmaceutical composition of the present invention to act on the target site. To this end, a composition suitable for use in the present invention comprises a formulation comprising the active ingredient of the present invention in combination with a pharmaceutically acceptable carrier. The growth factor in the composition of the present invention is preferably contained at a concentration of 0.000001% to 0.01%. As used herein, the term "pharmaceutically acceptable" has the meaning of being miscible with other components of the formulation without harming a person receiving the same.

In the present invention, the composition preferably comprises 0.000001% to 0.01% of the growth factor and 0.00001% to 10% of the silk protein, and more preferably 0.01 to 2 ppm of the growth factor and 0.0001% to 1% of the silk protein.

In the composition, if the growth factor is contained in an amount of 10 ppm or more, an improvement in the effect of treating hair loss cannot be expected despite the additional amount, whereas if the growth factor is contained in an amount less than 0.00001%, an effect of treating hair loss cannot be expected. Also, if the silk protein is contained in an amount of 10% or more, an improvement in the effect of treating hair loss cannot be expected despite the additional amount, whereas if the silk protein is contained in an amount less than 0.001%, an effect of treating hair loss cannot be expected.

In another aspect, the present invention is directed to a pharmaceutical composition for promoting hair growth or inhibiting hair loss comprising VEGF, FGFα, FGFβ, TGF-β, IGF, KGF, and SCF as active ingredients.

The composition of the present invention may further comprise a silk protein.

In the present invention, the silk protein may be naturally derived, or be derived from a recombinant silk protein, and the silk protein may be a repeating unit peptide constituting a protein selected from the group consisting of dragline silk, elastin, silk fibroin, byssus, sericin, flagelliform silk, and collagen.

In the present invention, the silk protein may have a structure in which a silk repeating unit peptide having a glycine or serine content of 1% or more is repeated 1 to 160 times, and the silk protein may have a molecular weight of 5 to 300 kDa.

The composition of the present invention may be manufactured in a formulation selected from the group consisting of an essence, shampoo, cream, lotion, tonic, spray, aerosol, oil, solution, suspension, gel, and ointment.

In still another aspect, the present invention is directed to a cosmetic composition for promoting hair growth or inhibiting hair loss comprising a growth factor selected from the group consisting of VEGF, FGFα, FGFβ, TGF-β, IGF, KGF, and SCF as an active ingredient.

The composition of the present invention may further comprise a silk protein.

In the present invention, the silk protein may be naturally derived, or be derived from a recombinant silk protein, and the silk protein may be a repeating unit peptide constituting a protein selected from the group consisting of dragline silk, elastin, silk fibroin, byssus, sericin, flagelliform silk, and collagen.

In the present invention, the silk protein may have a structure in which a silk repeating unit peptide having a glycine or serine content of 1% or more is repeated 1 to 160 times, and the silk protein may have a molecular weight of 5 to 300 kDa.

In yet another aspect, the present invention is directed to a cosmetic composition for promoting hair growth or inhibiting hair loss comprising VEGF, FGFα, FGFβ, TGF-β, IGF, KGF, and SCF as active ingredients.

The composition of the present invention may further comprise a silk protein.

In the present invention, the silk protein may be naturally derived, or be derived from a recombinant silk protein, and the silk protein may be a repeating unit peptide constituting a protein selected from the group consisting of dragline silk, elastin, silk fibroin, byssus, sericin, flagelliform silk, and collagen.

In the present invention, the silk protein may have a structure in which a silk repeating unit peptide having a glycine or serine content of 1% or more is repeated 1 to 160 times, and the silk protein may have a molecular weight of 5 to 300 kDa.

The composition of the present invention is topically applied onto the portion of the scalp to receive hair loss treatment for about 1 week to about 8 weeks, thereby promoting, inducing and stimulating hair growth and/or reducing hair loss.

In still yet another aspect, the present invention is directed to a method of promoting hair growth or inhibiting hair loss comprising administering a growth factor selected from the group consisting of VEGF, FGFα, FGFβ, TGF-β, IGF, KGF, and SCF.

In further aspect, the present invention is directed to the use of a growth factor selected from the group consisting of VEGF, FGFα, FGFβ, TGF-β, IGF, KGF, and SCF for the promotion of hair growth or inhibition of hair loss.

In still a further aspect, the present invention is directed to the use of a growth factor selected from the group consisting of VEGF, FGFα, FGFβ, TGF-β, IGF, KGF, and SCF for the preparation of a therapeutic agent for the promotion of hair growth or inhibition of hair loss.

The composition according to the invention is typically administered in a topical form. The formulation suitable for topical administration includes a liquid or semi-liquid formulation such as a lotion, emulsion, cream, ointment, liniment, spray, aerosol, oil, paste, gel, tonic, solution, or suspension. In order to manufacture such a hair growth formulation, various components may be mixed and dissolved or the mixture may be kneaded and then formulated using any device and method commonly used or well-known in the pharmaceutical and/or cosmetic fields [Reference: Remington's Pharmaceutical Science, 15th Edition, 1975, Mack Publishing Company, Easton, Pennsylvania 18042 (Chapter 87: Blaug, Seymour)]. A preferred formulation is a gel, ointment, lotion, or cream.

For ointments, plasma or serum is suspended or dissolved in any one or a mixture of two or more selected from among the following components: mineral oil, paraffin, paraffin oil, white petrolatum, propylene glycol, polyoxyethylene, polyoxypropylene, glycerin, stearyl alcohol, emulsifying wax, cetanol, sodium lauryl sulfate, ethyl or butyl paraoxybenzoate, saline, and water. For lotions or creams, plasma or serum is suspended or dissolved in any one or a mixture of two or more selected from among the following components: mineral oil, sorbitan monostearate, polysorbate 60, petrolatum, lanolin, saline, cetyl ester wax, cetearyl alcohol, 2-octyldodecanol, benzyl alcohol, and water. In addition to the above components, the formulation of the present invention may comprise at least one additional component selected from among diluents, buffers, flavoring agents, binders, surfactants, thickeners, lubricants, preservatives, pH adjusters, disinfecting agents, antioxidants, emulsifiers, stabilizers, fragrances, colorants, and the like.

The dose of the composition according to the present invention has to be appropriately determined in consideration of gender, age, the extent of hair loss, hair condition, and the like. A single dose for application to the scalp of normal adults is about 0.1 to about 5 mg/cm² per day.

Hereinafter, the present invention will be described in more detail with reference to examples. However, it will be obvious to those skilled in the art that these examples are provided only for illustration of the present invention and should not be construed as limiting the scope of the present invention.

### Example 1. Cell scratch assay using growth factor

It was confirmed whether a cell scratch was healed by treatment of growth factors (VEGF, FGFα, FGFβ, TGF-β, IGF, KGF, and SCF).

NIH3T3 cells (ATCC CRL-1658™), which are fibroblasts, were used, and when the cells grew to 80% or more in a DMEM + bovine serum + pen-strep (antibiotic) medium (containing 10% serum, 100X concentration of penicillin-streptomycin added at 1% of the total volume) under 5% CO₂ (Thermo Fisher BB15), scratching was performed using a pipette tip.

After scratching, the cells were washed two times with a PBS buffer, after which 10 mL of a culture medium was added thereto. With regard to the culture medium that was added, a growth medium (DMEM + bovine serum + pen-strep) was added for a positive control group, a medium in which only the antibiotic pen-strep was added to DMEM was used for a negative control group, and for experimental groups, a DMEM + pen-strep medium containing 0.0001% of each of VEGF, FGFα, FGFβ, TGF-β, IGF, KGF, and SCF growth factors was used.

As growth factors, liquid growth factors at a concentration of 100 ppm were purchased from PnP Biopharm and used.

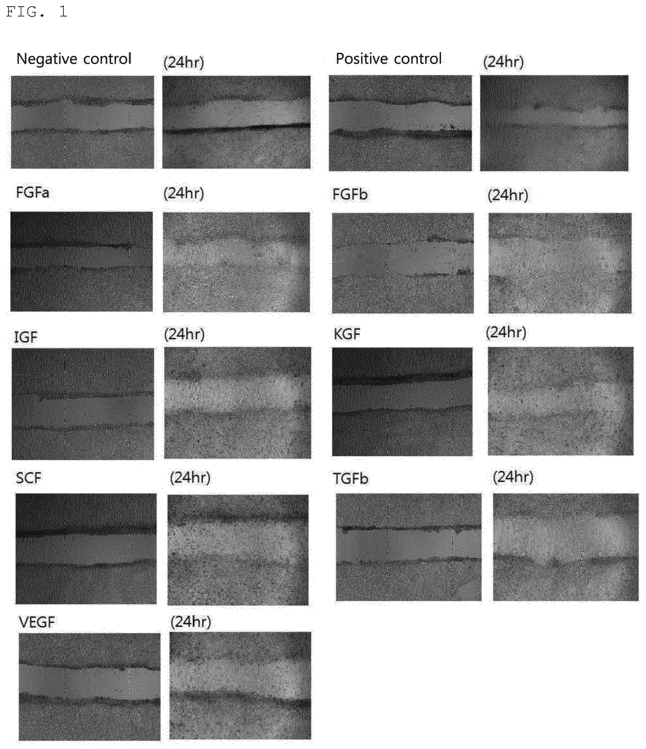

As a result, as shown in FIG. 1, in the group administered with each of VEGF, FGFα, FGFβ, TGF-β, IGF, KGF, and SCF, it was confirmed that all of them exhibited a fast recovery from scratch compared to the control groups after 24 hours.

Moreover, a scratch assay was performed using a culture containing a silk protein alone or both a silk protein and a growth factor.

As the silk protein, a recombinant silk protein was used, and the recombinant silk protein was manufactured using the method described in the existing patent (Korean Patent No. 10-1317420 B1).

A high-molecular-weight recombinant silk or silk-like protein having a structure in which a peptide having the sequence of SEQ ID NO: 1 below is repeated 48 times was used.

SEQ ID NO: 1:
NH₂-SGRGGLGGQGAGMAAAAAMGGAGQGGYGGLGSQGT-COOH

For production, fed-batch fermentation was carried out, and the target protein was extracted using dialysis.

The same method as the scratch assay for the growth factor was performed, and the silk protein was used at a concentration of 0.05%.

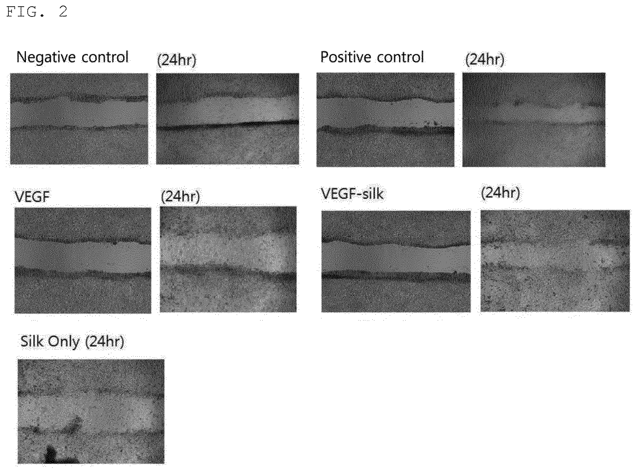

As a result, as shown in FIG. 2, it was confirmed that when VEGF and the silk protein were added together, a scratch recovery was superior compared to the control group using the silk protein alone and the control group using VEGF alone.

### Example 2. Confirmation of hair growth effect in hair-loss mouse model

In order to completely depilate the backs of eight 5-week-old white mice (400-500 g, Sprague-Dawley type), the hair was shaved, and 20-30 g of a mouse removal cream (Veet) was then applied to each mouse, and after 5 minutes, the mouse removal cream was wiped off, so the hair was completely removed. A hair-loss mouse model was manufactured by applying 40 mg of testosterone/2 g of gel/mouse onto the area from which hair had been removed twice daily in the morning and evening for 2 weeks.

A basic essence (hair tonic from Medicos Biotech, Table 2) was added with the growth factors (VEGF, FGFα, FGFβ, TGF-β, IGF, KGF, and SCF) and/or the silk protein as shown in Table 1 below, and was then applied to the area of each mouse from which hair had been removed at 10 ml/mouse/day for 1 week.

**[Table 1]**

| Groups | Application components |
|---|---|
| Control group | Basic essence |
| 2.5% p.p. | 2.5% serum |
| 1.5 ppm | 1.5 ppm growth factor mixture |
| 1 ppm | 1 ppm growth factor mixture |
| 0.5 ppm | 0.5 ppm growth factor mixture |
| 1 ppm + silk | 1 ppm growth factor mixture + 0.05% silk protein |
| 0.5 ppm + silk | 0.5 ppm growth factor mixture + 0.05% silk protein |

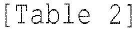

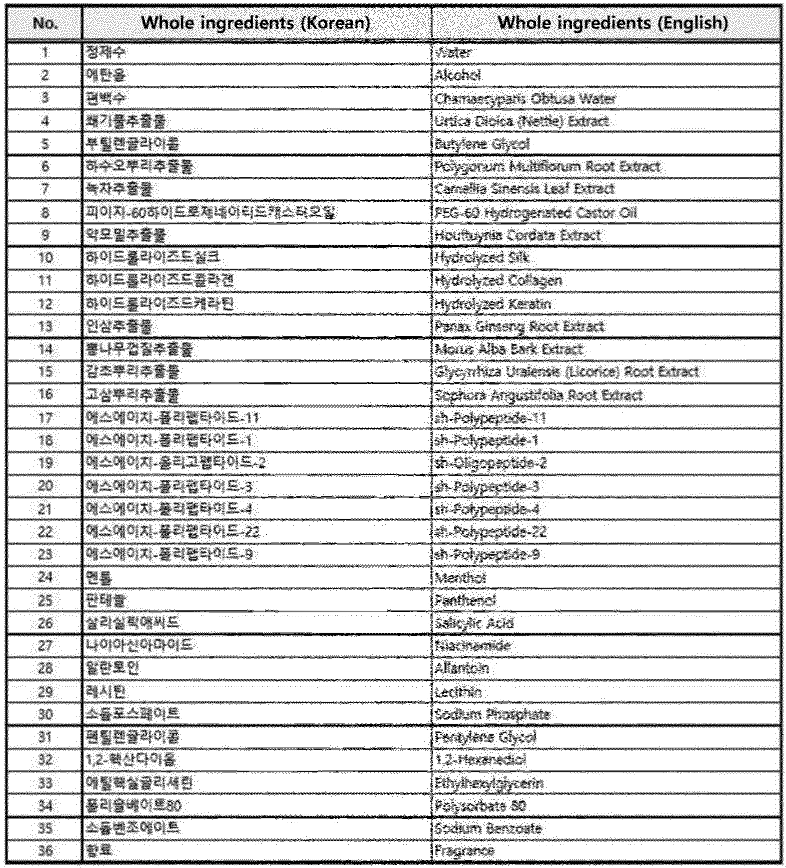

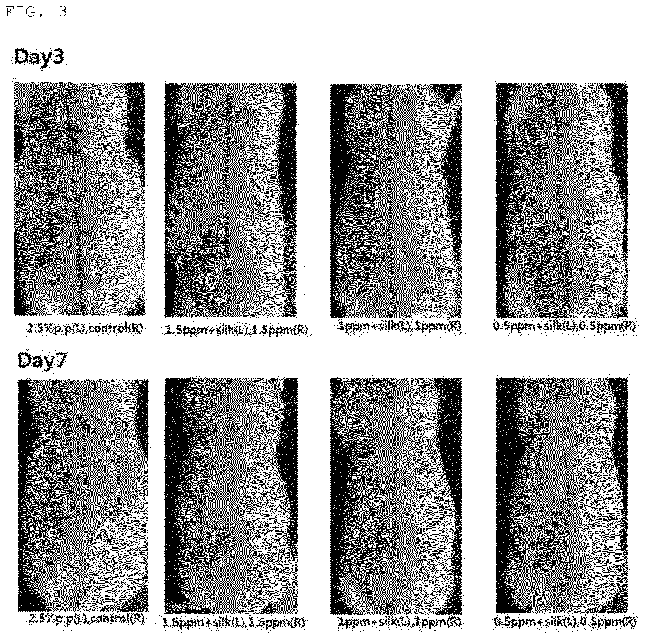

Based on the results of observation of the mice after 7 days, as shown in FIG. 3, the hair growth effect was confirmed in the mice treated with seven growth factors compared to the control groups, and was also confirmed in the mice treated with seven growth factors and silk protein together, indicating that the higher the concentration of the growth factor that was used, the better the hair grew, and also that the hair grew much better in the group treated in combination with the silk protein.

### Industrial Applicability

According to the present invention, there is an effect of promoting hair growth and preventing hair loss by regulating the hair cycle of patients with hair loss, without causing side effects such as skin irritation and the like.

Although specific embodiments of the present invention have been disclosed in detail as described above, it will be obvious to those of ordinary skill in the art that the description is merely of preferable exemplary embodiments and is not to be construed as limiting the scope of the present invention. Therefore, the substantial scope of the present invention will be defined by the appended claims and equivalents thereof.

### Sequence Free Text

An electronic file is attached.

## Claims

1. A pharmaceutical composition for promoting hair growth or inhibiting hair loss comprising a growth factor selected from the group consisting of VEGF, FGFα, FGFβ, TGF-β, IGF, KGF, and SCF as an active ingredient.

2. The pharmaceutical composition according to claim 1, wherein the composition comprises at least two growth factors selected from the group consisting of VEGF, FGFα, FGFβ, TGF-β, IGF, KGF, and SCF.

3. The pharmaceutical composition according to claim 1, further comprising a silk protein.

4. The pharmaceutical composition according to claim 3, wherein the silk protein is naturally derived, or is derived from a recombinant silk protein.

5. The pharmaceutical composition according to claim 3, wherein the silk protein is a repeating unit peptide constituting a protein selected from the group consisting of dragline silk, elastin, silk fibroin, byssus, sericin, flagelliform silk, and collagen.

6. The pharmaceutical composition according to claim 3, wherein the silk protein has a structure in which a peptide having a glycine or serine content of 1% or more is repeated 1 to 160 times.

7. The pharmaceutical composition according to claim 3, wherein the silk protein has a molecular weight of 5 to 300 kDa.

8. The pharmaceutical composition according to claim 1, wherein the composition is in a formulation selected from the group consisting of an essence, shampoo, cream, lotion, tonic, spray, aerosol, oil, solution, suspension, gel, and ointment.

9. A pharmaceutical composition for promoting hair growth or inhibiting hair loss comprising VEGF, FGFα, FGFβ, TGF-β, IGF, KGF, and SCF as active ingredients.

10. The pharmaceutical composition according to claim 9, further comprising a silk protein.

11. The pharmaceutical composition according to claim 10, wherein the silk protein is naturally derived, or is derived from a recombinant silk protein.

12. The pharmaceutical composition according to claim 10, wherein the silk protein is a repeating unit peptide constituting a protein selected from the group consisting of dragline silk, elastin, silk fibroin, byssus, sericin, flagelliform silk, and collagen.

13. The pharmaceutical composition according to claim 10, wherein the silk protein has a structure in which a peptide having a glycine or serine content of 1% or more is repeated 1 to 160 times.

14. The pharmaceutical composition according to claim 10, wherein the silk protein has a molecular weight of 5 to 300 kDa.

15. The pharmaceutical composition according to claim 9, wherein the composition is in a formulation selected from the group consisting of an essence, shampoo, cream, lotion, tonic, spray, aerosol, oil, solution, suspension, gel, and ointment.

16. A cosmetic composition for promoting hair growth or inhibiting hair loss comprising a growth factor selected from the group consisting of VEGF, FGFα, FGFβ, TGF-β, IGF, KGF, and SCF as an active ingredient.

17. The cosmetic composition according to claim 16, wherein the composition comprises at least two growth factors selected from the group consisting of VEGF, FGFα, FGFβ, TGF-β, IGF, KGF, and SCF.

18. The cosmetic composition according to claim 16, further comprising a silk protein.

19. The cosmetic composition according to claim 18, wherein the silk protein is naturally derived, or is derived from a recombinant silk protein.

20. The cosmetic composition according to claim 18, wherein the silk protein is a repeating unit peptide constituting a protein selected from the group consisting of dragline silk, elastin, silk fibroin, byssus, sericin, flagelliform silk, and collagen.

21. The cosmetic composition according to claim 18, wherein the silk protein has a structure in which a peptide having a glycine or serine content of 1% or more is repeated 1 to 160 times.

22. The cosmetic composition according to claim 18, wherein the silk protein has a molecular weight of 5 to 300 kDa.

23. A cosmetic composition for promoting hair growth or inhibiting hair loss comprising VEGF, FGFα, FGFβ, TGF-β, IGF, KGF, and SCF as active ingredients.

24. The cosmetic composition according to claim 23, further comprising a silk protein.

25. The cosmetic composition according to claim 24, wherein the silk protein is naturally derived, or is derived from a recombinant silk protein.

26. The cosmetic composition according to claim 24, wherein the silk protein is a repeating unit peptide constituting a protein selected from the group consisting of dragline silk, elastin, silk fibroin, byssus, sericin, flagelliform silk, and collagen.

27. The cosmetic composition according to claim 24, wherein the silk protein has a structure in which a peptide having a glycine or serine content of 1% or more is repeated 1 to 160 times.

28. The cosmetic composition according to claim 24, wherein the silk protein has a molecular weight of 5 to 300 kDa.
